# EUROPEAN PATENT SPECIFICATION

(11) **EP 1 976 434 B1**
(45) Date of publication and mention of the grant of the patent: **13.01.2016**
(21) Application number: 06733894.7
(22) Date of filing: 25.01.2006
(51) Int. Cl.: A61B 5/145, A61B 5/151, A61B 5/15

(54) **LANCET SENSOR ASSEMBLY AND METER**
LANZETTEN-SENSORANORDNUNG UND MESSGERÄT
ENSEMBLE CAPTEUR À LANCETTE ET COMPTEUR

(43) Date of publication of application: 08.10.2008
(73) Proprietor: NOVA BIOMEDICAL CORPORATION, Waltham, MA 02454-9141 (US)
(72) Inventor: FOWLER, James, Brewster, Massachusetts 02631 (US); DAGGETT, Robert, Chelmsford, Massachusetts 01824 (US); O'CONNELL, Garland, Newtonville, Massachusetts 02162 (US); SIDWELL, James S., Boxborough, Massachusetts 01719 (US); ROBBINS, Avi M., Marietta, Georgia 30067 (US); RUF, Chris, Marietta, Georgia 30067 (US); STOUT, Jeffrey T., Smyrna, Georgia 30080 (US)
(74) Representative: von Hellfeld, Axel
(86) International application number: PCT/US2006/002668
(87) International publication number: WO 2007/086843

(56) References cited:
- EP-A- 1 285 629
- WO-A-03/015627
- WO-A-2005/046477
- WO-A-2005/107595
- WO-A2-02/05872
- US-A1- 2003 050 656
- US-A1- 2004 225 311
- US-A1- 2005 149 090
- US-A1- 2005 277 850
- US-B1- 6 530 892

## Description

### BACKGROUND OF THE INVENTION

### 1. Field of the Invention

The present invention relates generally to testing body fluids. Particularly, the present invention relates to a. lancet used for obtaining a sample of body fluid for testing. More particularly, the present invention relates to a lancet and test strip combination. Still more particularly, the present invention relates to a diagnostic system incorporating a lancet and test strip combination and an electronic meter.

### 2. Description of the Prior Art

The examination of blood samples in clinical diagnostics enables the early and reliable recognition of pathological states as well as a specific and well-founded monitoring of physical condition. Lancets and lancet devices enable blood sample collection especially for home monitoring by diabetics.

A blood sugar level that is either too high or low can lead to adverse physical consequences for a diabetic. Personal blood sugar determination is important for diabetics to aid in controlling and maintaining blood sugar levels with the use of insulin and other medications. A lancet is used to pierce the skin (usually a finger) and produce a small blood sample. Lancing the skin is painful. For diabetics who are required to test their blood sugar level several times a day, lancing several times a day is a painful but necessary procedure. The blood sample is then placed on a test strip for analysis and the blood glucose level is read by a blood glucose meter. Various devices have been devised for lancing the skin of a user as well as combination devices that include lancets and analytical device.

U.S. Patent No. 6,620,112 (2003, Klitmose) discloses a disposable lancet combined with a reagent carrying strip which carries a reagent that indicates the concentration of a blood component in a blood sample placed in contact with the strip The reagent carrying strip is connected to the lancet, e.g. by molding. One end of the lancet is sharpened for piercing the skin. The strip is sheet-like and has a first side and a second side, which sides are both accessible for the user, such that the reagent carrying strip can be inserted into a blood glucose meter. A weakened tear line is provided at a connection between the lancet and an edge of the reagent carrying strip so that the reagent carrying strip may be easily disconnected from the lancet.

U.S. Patent Application Publication No. US2003/0050573 (Kuhr et al.) discloses an analytical device containing a lancet comprising a lancet needle and a lancet body, the lancet needle being movable relative to the lancet body and the lancet body being composed, at least in the area of the tip of the lancet needle, of an elastic material in which the tip of the lancet needle is embedded, and an analytical test element which is permanently connected to the lancet body. In addition the invention concerns an analytical device containing a lancet comprising a lancet needle and lancet body which is in the form of a hollow body in the area of the tip of the lancet needle and surrounds the tip of the lancet needle, the lancet needle being movable relative to the lancet body and the hollow body being composed at least partially of an elastic material, and an analytical test element which is permanently connected to the lancet body.

U.S. Patent No. 6,607,658 (2003, Heller et al.) discloses an analyte measurement device includes a sensor strip combined with a sample acquisition device to provide an integrated sampling and measurement device. The sample acquisition device includes a skin piercing member such as a lancet attached to a resilient deflectable strip which may be pushed to inject the lancet into a patient's skin to cause blood flow. The resilient strip is then released and the skin piercing member retracts.

U.S. Patent Application Publication No. 2002/0130042 (Moerman et al.) discloses an apparatus having a meter unit, a lancet and an electrochemical sensor. The meter is reusable while the lancet and the electrochemical sensor are incorporated into assemblies intended for single use. The meter has a housing within which a lancet is engaged with a mechanism for moving the lancet, a connector disposed within the housing for engaging an electrochemical sensor specific for the analyte, and a display operatively associated with a connector for displaying the amount of the analyte to the user.

U.S. Patent Application Publication No. 2002/0082522 (Douglas et al.) discloses a device and method for lancing a patient, virtually simultaneously producing and collecting a small fluid sample from the body. The device includes a lancing needle, drive mechanism, kneading or vibration mechanism, optional suction system, and sample ejection mechanism.

Document US 2005/277850 also discloses a similar lancet sensor assembly.

A disadvantage of the above prior art is that each of the lancets are rigid and rely solely on the spring action of a firing mechanism to retrieve the lancet after firing or, in the case of the Heller device, the specimen piercing speed of the lancet is uncontrolled and depends on the quickness of the user. Further, the prior art that provides for shallow depth penetration of the lancet generally includes a sophisticated system to knead the surrounding lanced area by ultrasonic action, piezo-electric or mechanical oscillation to stimulate the blood flow from the wound to draw the blood into a pumping system. It should also be noted that none of the prior art lancet sensor combination devices are currently available in the marketplace, which indicates that the prior art devices do not provide a reasonable, cost-effective, useful, and workable system for a lancet sensor combination test strip and meter.

Currently available, prior art, blood glucose meters include those known as the Accu-Chek^{®} Aviva system by Roche Diagnostics, the One-Touch^{®} system by LifeScan, the Glucometer^{®} DEX system by Bayer, the True Track^{®} system by Home Diagnostics, and the Freestyle^{®} system by Abbott. Although these meters advertise various advantages such as fast and reliable test results, small volume requirements and reduced pain systems, each of the currently available meters requires the use of a separate lancing device to obtain the blood sample from the patient. Some require the occasional use of a control reagent to calibrate the meter. All, however, require a patient to carry both the meter and the lancing device with the appropriate number of disposable test strips and lancets. None of these currently available meters, on the other hand, are capable of accepting a combination lancet sensor test strip or eliminating the need for a separate lancing device.

Therefore, what is needed is a lancet assembly that has an inherent return action upon piercing a specimen. What is further needed is a lancet assembly that can incorporate an analytical test strip. What is also needed is a test strip diagnostic, handheld meter that is capable of driving a lancing device and electronically testing a blood sample. What is still needed is a diagnostic, handheld meter that is usable with a lancet sensor test strip combination.

### SUMMARY OF THE INVENTION

It is an object of the present invention to provide a lancet assembly that has an inherent return action upon piercing a specimen. It is another object of the present invention to provide a lancet assembly capable of incorporating an analytical test strip forming a disposable, integrated unit. It.is a further object of the present invention to provide a diagnostic, handheld meter that is capable of driving a lancing device and electronically testing a blood sample. It is still another object of the present invention to provide a diagnostic, handheld meter that is usable with a lancet sensor test strip combination. It is yet another object of the present invention to provide a blood glucose diagnostic system that provides fast, reliable results and is virtually pain free when obtaining a blood sample.

The present invention achieves these and other objectives by providing each of the following: a lancet assembly having at least a lancet and an elongated carrier for holding the lancet; a lancet sensor combination test strip assembly; a diagnostic, handheld meter incorporating measuring circuitry and a lancing mechanism for use with a lancet sensor combination test strip assembly; and a portable glucose test system that includes a lancet sensor strip assembly and a diagnostic, handheld meter.

The lancet of the lancet assembly includes a lancet body, a lance extending from the lancet body on one end, a sinuous portion extending from the lancet body on an opposite end, and a drive wing extending outwardly from a side of the lancet body. The lance may be flat, round or any shape commonly used as a lancet for lancing the skin. The sinuous portion has a distal end with an anchor portion. The lancet body may optionally include a second drive wing extending outwardly from the opposite side of the first drive wing.

The elongated carrier includes a lancet receiving recess, an open end that is also the needle end, a closed end, a first side, a second side, a first side opening, an optional second side opening, an anchor mechanism, and an assembly retaining mechanism. The first and second sides include supporting edges for supporting an optional lancet assembly cover or a sensor test strip. The assembly retaining mechanism is a plurality of tabs that can be bent over the recess or deformed to retain the lancet within the elongated carrier. The first side opening is elongated to allow the drive wing to extend outside of the elongated carrier in position to cooperate with a lancet driver. The elongated side opening allows the drive wing, lancet body and lance to slide between a retracted position and an extended position. The anchor mechanism engages the anchor portion of the lancet to prevent the distal end of the sinuous portion from moving when the drive wing is engaged to cause the lancet to slide to the extended position. The elongated carrier may optionally include side notches or slots near the closed end to enhance retention of the lancet assembly when inserted into a meter or lancing device.

In one embodiment, the lancet carrier is made of metal and has a plurality of bendable tabs and an optional bottom groove. The metal elongated carrier is stamped, cut and bent to the desired shape. The optional bottom groove is formed by stamping and creates a rib along the outside surface of the bottom. The optional bottom groove/rib provides not only stability to the elongated carrier but also serves as a guide when inserting the lancet assembly into a meter/lancing device. In another embodiment, the lancet carrier is made of plastic that allows for molding/thermoforming the lancet carrier.

In both lancet assembly embodiments, the elongated carrier may optionally include one or more wing guards that extend away from the side of the elongated carrier in the vicinity of the elongated side opening where the drive wing is located. The wing guard protects the drive wing of the lancet from being inadvertently hit when being handled by a user and/or inserted to a meter or lancing device. Furthermore, the depth of the recess in the lancet carrier is deeper than the thickness of the lancet so that the lancet body can freely move the lancet tip out of the needle end from a retracted position to an extended position and back to the retracted position.

In another embodiment, the lancet assembly may optionally include a test strip attached to the top side of the lancet carrier. The test strip typically includes a sample fluid entrance port, a sample chamber with at least one sensor and a sample vent hole. Electrical contacts are situated at the opposite end of the test strip for connecting to a meter.

The diagnostic, handheld meter has measuring circuitry, lancing driver assembly components and a test strip port incorporated into a meter housing. The measuring circuitry is preferably an electrochemical measuring circuit designed for using a particular electrochemical measuring method such as, for example, amperometric, coulometric, potentiometric, voltammetric, or other electrochemical techniques. A lancet sensor test strip socket is connected to the measuring circuitry to provide an electrical connection between the sensor strip and the measuring circuitry. The lancing driver assembly components include a lancet driver, a lancet trigger, a test strip receiver platform, and an optional lancing depth control.

The lancet trigger is an asymmetrical trigger. The asymmetrical trigger includes a trigger body that is typically secured to the meter housing, a lancet driver piston release positioned near the base of the trigger body, and a user interface, positioned on a trigger arm that extends outwardly from the top of the trigger body. The user interface is located along the central axis of the meter, which also coincides with the central axis of the disposable lancet sensor test strip. It is the asymmetrical design of the trigger relative to the trigger body that allows the user interface to be located along the central axis of the meter and test strip port providing the user with easy and comfortable access to the firing trigger regardless whether the user is right-handed or left-handed. Because of the user interface's position along the central axis of the meter and the test strip port, it makes the lancing procedure easy and comfortable for the user. No other prior art device has this structure.

The test strip receiver platform supports the disposable lancet sensor test strip when it is inserted into the meter. The test strip receiver platform has two platform sides, a proximal end and a distal end. A portion of a first platform side at the proximal end is exposed at the test strip port. This makes it easy for the user to load and insert a disposable lancet sensor test strip. The distal end of the receiver platform includes a cross support with guide hooks on each end for cooperating with a charging member of the lancet driver. The first platform side includes a driver slot through which a lancet driver surface extends for engaging the drive wing of the lancet sensor test strip. The first platform side may also include a test strip guide groove when the test strip incorporates a mating guide rib. A second platform side slidingly supports a driver piston of the lancet driver.

The lancet driver includes a piston driver, a driver charging member, a piston drive spring, a pair of piston return springs, and a pair of charging member return springs. The piston driver has a piston body with a lancet driver surface located near a drive wing end, a drive spring recess for receiving the piston drive spring and a pair of return spring arms that extend away from the driver body. As mentioned above, the driver piston is slidingly supported by the second platform side of the test strip receiver platform with the lancet driver surface extending through the driver slot to the first platform side for engaging with the drive wing of the test strip. The piston drive spring is secured on one end within the drive spring recess while the other end contacts a central portion of the cross support of the test strip receiver platform. The central portion of the cross support acts as a stop surface for the drive spring when the driver piston is loaded into the "armed" position. The proximal end of the driver piston has a driver piston holding surface that cooperates with the driver piston release of the lancet trigger to hold the driver piston in the "armed" position until released by the user.

The driver charging member has a handle, a pair of parallel charging member rails that extend from the inside of the handle, charging member arms that extend perpendicularly from the inside of each of the charging member rails towards each other, a stop interface on one end of one of the charging member rails, and a pair of charging member return springs. Each of the charging member return springs connects on one end to the charging member rails and on the other end to the meter housing. Each of the charging member rails slidingly engages with one of the receiver platform side edges. The charging member arms also include a piston stop surface that is used to engage and arm the driver piston when the charging handle is pulled and to stop the sliding movement of driver piston when it is discharged from the armed position.

The meter housing may optionally include a lancing depth control. The lancing depth control has a detent side and a depth gauge side. The detent side includes a plurality of tabs extending out of the surface of the detent side with spaces between each of the plurality of tabs for receiving the detent. The tabs are relatively rigid but sufficiently flexible to allow the tabs to deflect and ride over the detent when the lancing depth control is changed. The depth gauge side has a charging member interface surface that is a gradual recessing surface that cooperatively engages the stop interface of the driver charging member to set the depth of lance penetration.

The portable glucose test system includes the handheld, portable meter capable of receiving a lancet sensor test strip, one or more disposable lancet sensor test strips, and an optional control solution. The handheld meter provides, in a single instrument, the dual functionality of driving a lance from the lancet sensor test strip to pierce a lancing site to obtain a sample and to perform the necessary electrochemical measurement steps to determine the concentration of glucose in the sample when it is added to the sample chamber of the sensor strip. No additional, separate lancing device is required to perform the lancing step.

### BRIEF DESCRIPTION OF THE DRAWINGS

FIGURE 1 is a top view of one embodiment of the lancet assembly of the present invention.
FIGURE 2 is a perspective view of the lancet carrier of the embodiment shown in Fig. 1.
FIGURES 3A and 3B are top views of the lancet of the present invention.
FIGURE 4 is a perspective view of one embodiment of the present invention showing a lancet sensor test strip with dual drive wings.
FIGURE 5 is a perspective view of the embodiment shown in Fig. 4 with a single drive wing.
FIGURE 6 is a perspective view of another embodiment of the lancet assembly of the present invention showing wing guards protecting the drive wings of the lancet.
FIGURE 7 is a perspective view of the elongated carrier of the lancet assembly in Fig. 6.
FIGURE 8 is an end view of the lancet assembly in Fig. 6 showing the guide rib on the bottom of the elongated carrier.
FIGURE 9A is a perspective view of another embodiment of the lancet carrier of the present invention.
FIGURE 9B is a top view of the lancet carrier shown in Fig. 9A.
FIGURE 9C is a bottom perspective view of the embodiment in Fig. 9A.
FIGURE 10 is a perspective view of one embodiment of the glucose measuring system of the present invention showing the portable meter with a disposable lancet sensor test strip inserted into the test strip port.
FIGURE 11 is a simplified, perspective view of the inside of the meter showing one embodiment of the lancing device components.
FIGURE 12A is a perspective view of the lancet trigger of the embodiment in Fig. 11.
FIGURE 12B is a perspective view of the test strip receiver platform of the embodiment in Fig. 11.
FIGURE 12C is a perspective view of the lancet driver of the embodiment in Fig. 11.
FIGURE 12D is a perspective view of the depth gauge control of the embodiment in Fig. 11.
FIGURE 13A is a front, perspective view of one embodiment of the optional depth gauge control of certain embodiments.
FIGURE 13B is a back, perspective view of the embodiment of the optional depth gauge control in Fig. 13A.
FIGURE 13C is a frontal view of the embodiment of the optional depth gauge control shown in Fig. 13A.

### DETAILED DESCRIPTION OF THE PREFERRED EMBODIMENT

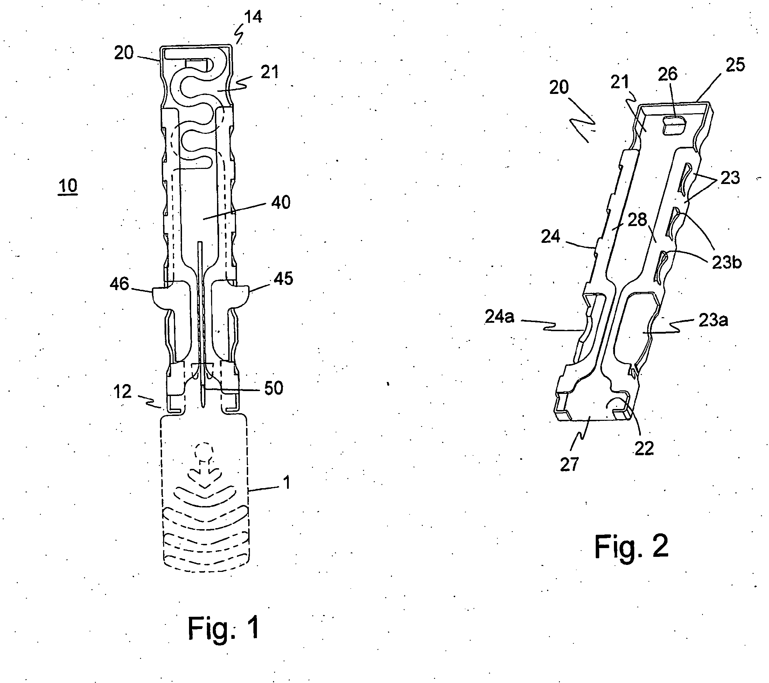

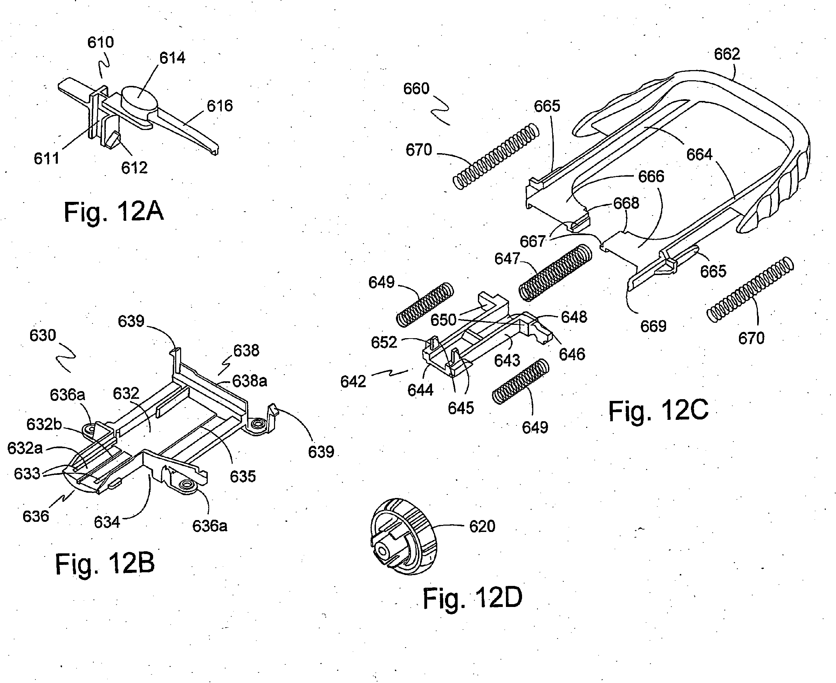

The preferred embodiment(s) of the present invention are illustrated in Figs. 1-11. Other embodiments are shown in Figs. 12-13. Figure 1 shows a lancet assembly 10 of the preferred embodiment of the present invention. Lancet assembly 10 includes a lancet carrier 20 and a lancet 40. Lancet carrier 20 includes a recess 21 that is configured to receive and contain lancet 40 when lancet assembly 10 is in a static state. Lancet assembly 10 has a needle end 12 through which lancet 40 protrudes and retracts during use and an anchor end 14. Lancet 40 has a first drive wing 45 and a second drive wing 46 that extend out the side of lancet carrier 20. A separate lancet cover (not shown) or a test strip (discussed later) may optionally be included, but is not necessary, with the lancet carrier 20. Lancet carrier 20 may be made of metal or a plastic material such as, for example, polyvinyl chloride, polycarbonate, polysulfone, nylon, polyurethane, cellulose nitrate, cellulose propionate, cellulose acetate, cellulose acetate butyrate, polyester, acrylic, and polystyrene. Lancet cover 1, which is represented by dashed lines, is not part of the invention but is used only to maintain the sterility of the lance 40 until it is used.

Figure 2 shows one embodiment of lancet carrier 20. In this embodiment, lancet carrier 20 is preferably made of sheet metal, which allows lancet carrier 20 to be stamped, cut and bent. Lancet carrier 20 has recess 21, a bottom 22, a first side 23, a second side 24, a closed end 25, an open end 27, a first side opening 23a, an optional second side opening 24a, an anchor member 26, and an assembly retaining mechanism 28. First side 23 and second side 24 have supporting edges 23b, 24b, respectively, for supporting a lancet assembly cover or a sensor test strip when either one is used as part of the assembly. Assembly retaining mechanism 28 are elongated tabs that are bent over recess 21 to retain lancet 40 and optional cover or test strip (not shown) when incorporated in the assembly. First side opening 23a and optional second side opening 24a are positioned to allow the outward extension of first drive wing 45 and optional second drive wing 46 through first side opening 23a and optional second side opening 24a, respectively, when lancet 40 is assembled in lancet carrier 20.

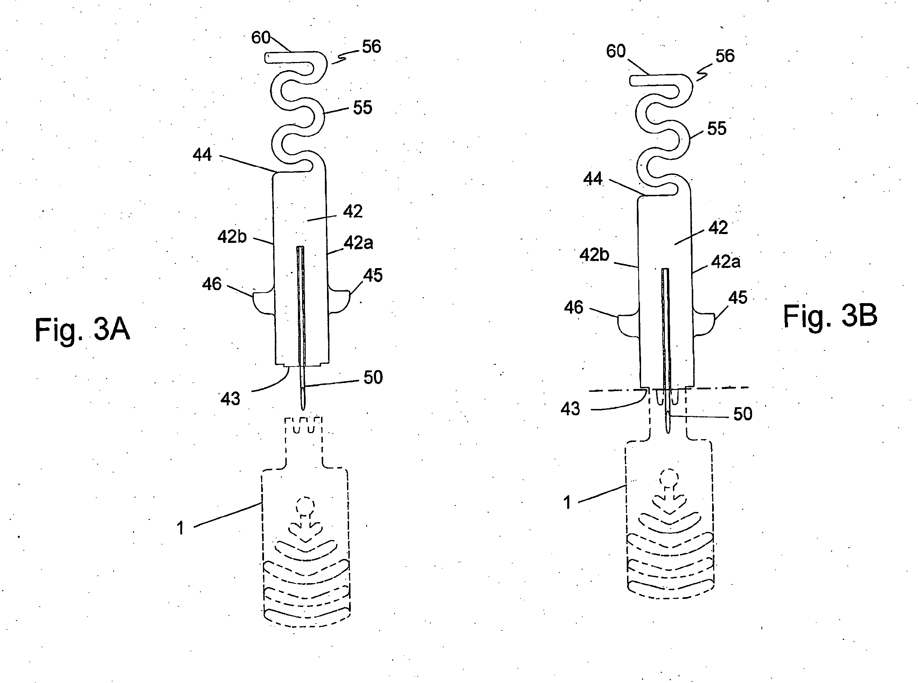

Figures 3A and 3B show an enlarged top view of lancet 40. Lancet 40 includes a lancet body 42, a lance 50, a sinuous portion 55, and an anchor portion 60 located at a sinuous portion distal end 56. Lancet body 42 has a lance end 43, a sinuous portion end 44, a first drive wing 45, and an optional second drive wing 46. First drive wing 45 and optional second drive wing 46 extend outwardly from a first side 42a and second side 42b, respectively, of lancet body 42. Lance 50 may be integrally made with lancet body 42 or may be a separate component that is fixedly attached to lancet body 42. A lancet driver (discussed later) cooperates with first drive wing 45 and optional second drive wing 46 to drive lancet 40 to an extended position. Lancet cover 1 is shown in Fig. 3B connected to lancet body 42 to protect lance 50 and maintain its sterility, and disconnected from lance body 42 in Fig. 3A to expose lance 50 for use.

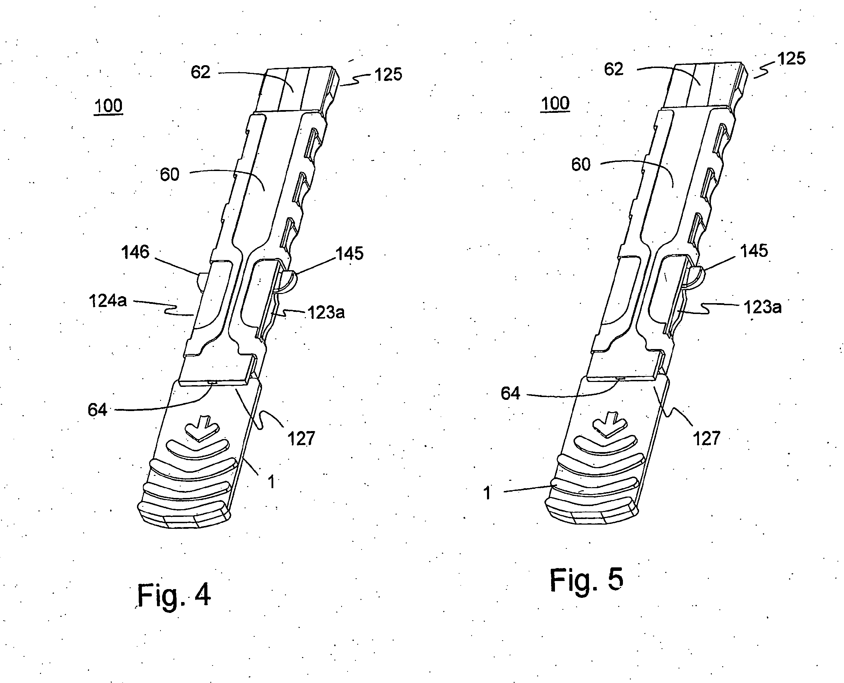

Turning now to Fig. 4, there is illustrated a lancet sensor assembly 100. Lancet sensor assembly 100 includes a lancet carrier 120, a lancet 140 with a first drive wing 145 extending outwardly from a first side opening 123a and a second drive wing 146 extending outwardly from a second side opening 124a, and a disposable sensor strip 60. Disposable sensor strip 60 has an electrical contact end 62 over closed end 125 and a sample receiving end 64 over open end 27. Fig. 5 illustrates another embodiment of lancet sensor assembly 100 but without the optional second drive wing 146. The remaining features are identical.

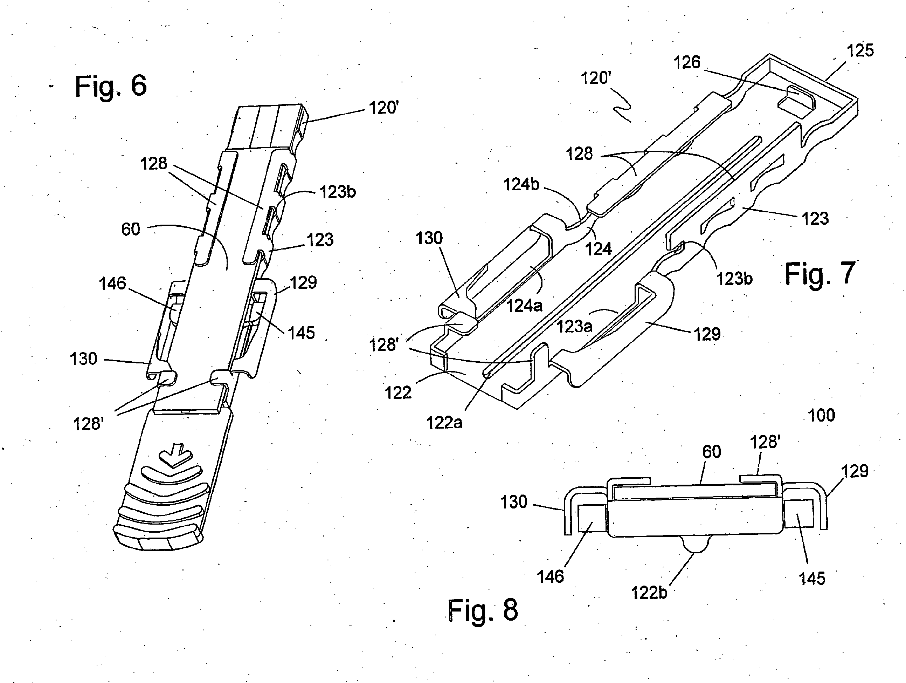

Fig. 6 illustrates another embodiment of lancet sensor assembly 100. Like the previously described embodiments, lancet sensor assembly 100 includes a lancet carrier 120', a lancet 140 and a sensor strip 60 combined in a single use, disposable unit. Lancet carrier 120' includes all of the features of lancet carrier 20 but with an additional feature. Lancet carrier 120' includes a first wing guard 129 and an optional second wing guard 130. First wing guard 129 extends over first drive wing 145. Retaining assembly mechanism includes tabs 128 behind first wing guard 129 and optional second wing guard 130 and front tabs 128' in front of first wing guard 129 and optional second wing guard 130. Tabs 128 and 128' are bent at about 90 degrees to the side walls 123, 124 to secure sensor strip 60 against supporting edges 123b, 124b. First wing guard 129 protects first drive wing 145 from being engaged or hit inadvertently with lancet sensor assembly 100 is being handled or inserted into a meter. Optional second wing guard 130 protects optional second drive wing 146.

Fig. 7 illustrates an enlarged perspective view of lancet carrier 120' to more clearly illustrate the features of lancet carrier 120'. As can be seen from Fig. 7, lancet carrier 120' includes another optional feature. The optional feature is a bottom groove 122a in bottom 122 that also creates a bottom rib 122b along the outside surface 122c of bottom 122. Bottom groove 122a acts as a carrier stiffener in the metal embodiment while bottom rib 122b also provides a guiding means when the lancet sensor assembly 100 is loaded into a handheld meter.

Fig. 8 is a front end view of lancet sensor assembly 100. Wing guards 129 and 130 wrap around the drive wings 145, 146 to protect the drive wings from being hit inadvertently when handled or inserted into a meter. Bottom rib 122b is also shown. It should be understood that bottom rib 122b does not have to be located along the central axis of lancet carrier 120' in order to obtain the benefits disclosed above.

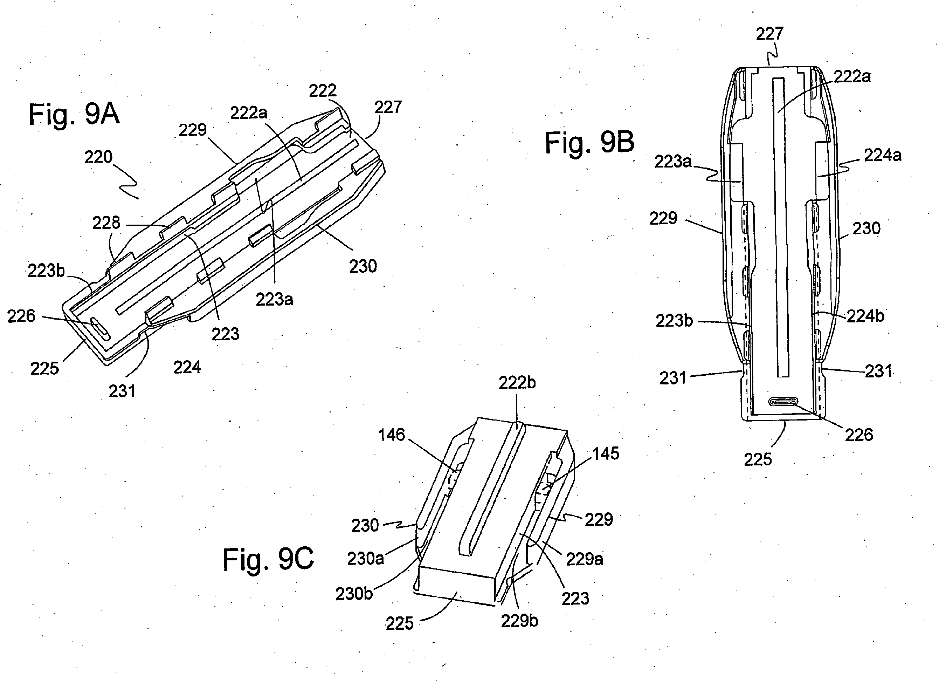

Turning now to Figures 9A, 9B and 9C, there is illustrated another embodiment of the lancet carrier. In this embodiment, lancet carrier 220 is preferably made of a plastic, thermoform material that can be easily molded. Like its metal counterpart, lancet carrier 220 includes a bottom 222, a first side 223, a second side 224, a closed end 225, an open end 227, a first side opening 223a, an optional second side opening 224a, an anchor member 226 located near closed end 225, and an assembly retaining mechanism 228. Assembly retaining mechanism 228 are preferably a plurality of deformable tabs that are deformed to retain the test strip/cover and lancet of the assembly. First side 223 and second side 224 have supporting edges 223b, 224b, respectively, for supporting a lancet assembly cover or a sensor test strip. Lancet carrier 220 also includes optional first wing guard 229 and optional second wing guard 230. Bottom 222 may include optional bottom groove 222a. In this embodiment, optional side notches 231 are included near closed end 225 to provide additional retention means for retaining the lancet sensor assembly when inserted into the meter. It should be understood that optional first and second wing guards 229, 230 may be any length and do not need to "wrap around" the drive wings. Wing guards 229, 230 need only extend over the drive wings sufficient to prevent the drive wings from being inadvertently hit by the user or the meter when being inserted. Fig. 9B illustrates a top view of lancet carrier 220 with the above described features.

Fig. 9C is a bottom, perspective view of lancet carrier 220. Lancet 220 has optional bottom rib 222b for use as a guiding means when inserting the lancet sensor assembly into a meter. Wing guards 229, 230 include wing driver channels 229b, 230b when optional wing guard side walls 229a, 230a are included. Wing driver channels 229b, 230b are formed between wing guard side walls 229a, 230a and first and second sides 223, 224. Drive wings 145, 146 are shown with dashed lines to provide their relative position when lancet 40 is assembled into lancet carrier 220. Wing driver channels 229b, 230b can also be used as guiding means when inserting the lancet sensor assembly into a meter.

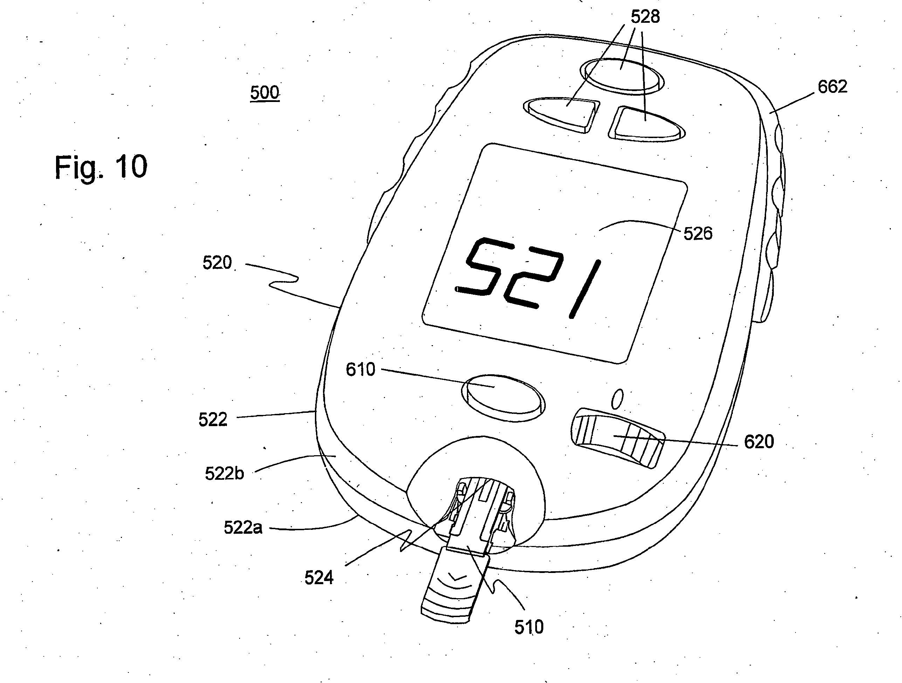

Turning now to Figure 10, there is illustrated a blood glucose test system 500. Test system 500 includes a lancet sensor strip assembly 510 and a handheld, portable, electrochemical measuring instrument/meter 520. Lancet sensor strip assembly 510 is similar to the previously described strip assemblies. Portable meter 520 includes a meter housing 522 with a housing body 522a and a housing cover 522b, a test strip socket 524, an electronic measuring circuit (not shown), a lancet driver charging system handle 662, a display 526, a plurality of meter measurement controls 528, a lancet trigger 610, and an optional lancing depth control 620. Meter 520 is typically battery powered for portability.

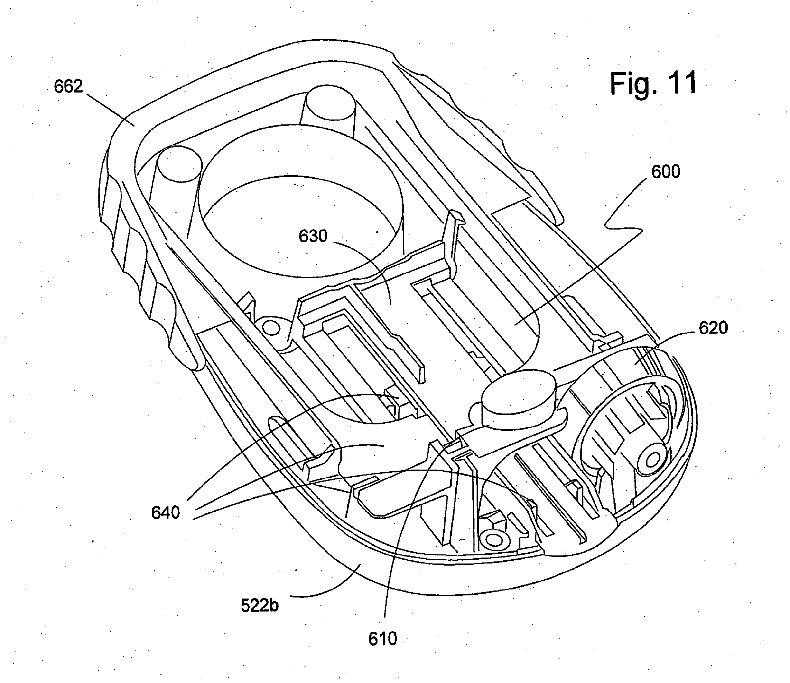

Figure 11 is an enlarged, simplified, perspective view of the inside of meter 520 with the housing cover 522b removed showing only the unique lancet driver assembly components 600 within housing body 522a for clarity. Lancet driver assembly components 600 include the lancet trigger 610, the optional lancing depth control 620, a test strip receiver platform 630, and a lancet driver 640.

Figures 12A-D show each of the lancet driver assembly components 600 separated for clarity. Fig. 12A is a perspective view of lancet trigger 610. Lancet trigger 610 is an asymmetrical trigger having a trigger body 611, a drive piston release 612 positioned near the base of trigger body 611, and a user interface 614 positioned on a trigger arm extension 616 that extends outwardly from the top of trigger body 611. When assembled, user interface 614 is located along the central axis of the meter 520, which coincides with the central axis of lancet sensor strip assembly 590.

Fig. 12B is a perspective view of test strip receiver platform 630. Test strip receiver platform 630 has a first platform side 632, a second platform side 634, a platform proximal end 636, and a platform distal end 638. First platform side 632 includes a test strip support surface 632a beginning at platform proximal end 636 for a pre-determined distance toward platform distal end 638. Test strip support surface 632a includes an optional guide groove 632b configured for receiving optional bottom rib 22b or 222b of lancet carrier 20, 200, respectively. Platform proximal end 636 is supported at test strip port 524 of meter 520 by a pair of proximal end legs 636a. Distal platform end 638 includes a cross support 638a with a guide hook 639 on each end. Test receiver platform 630 also includes at least one driver slot.633 parallel to the guide groove 632b and an elongated guide surface 635 for sliding cooperation with driver charging member 660.

Fig. 12C is a perspective view of lancet driver 640. Lancet driver 640 includes a driver piston 642 and a driver charging member 660. Driver piston 642 has a piston body 643 with a drive wing end 644 having at least one lancet driver surface 645, a drive spring recess 646, a pair of return spring arms 648 extending away from and perpendicular to driver body 643, a piston drive spring 647, and a pair of piston return springs 649. Driver piston 642 is positioned adjacent second platform side 634 with lancet driver surface 645 extending through driver slot 633 of test strip receiver platform 630 for engagement with drive wing 145 of lancet 40. Driver piston 642 also includes a driver piston holding surface 652 that cooperates with driver piston release 612 to hold driver piston 642 in an "armed" position. Drive spring recess 646 secures piston drive spring 647 on one end and the other end of piston drive spring 647 contacts a central portion of cross support 638a of test receiver platform 630. A piston return spring 649 is positioned on each side of piston body 643 between proximal end legs 636a of test strip platform 630 and the return spring arms 648. Each return spring arm 648 includes a charging contact stop surface 650.

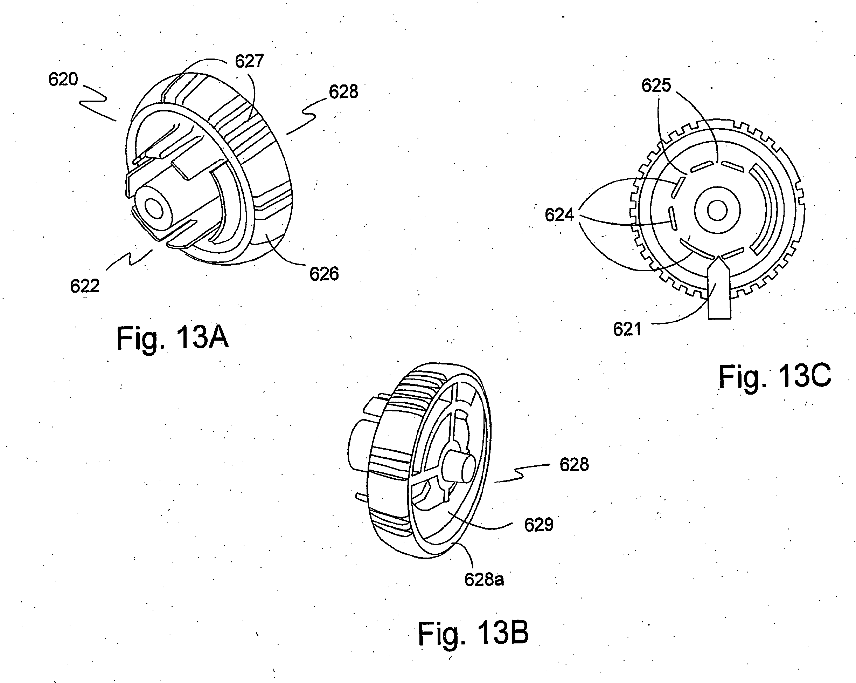

Driver charging member 660 has a charging system handle 662, a pair of parallel, charging member rails 664 extending from the inside of handle 662, charging member arms 666 located distally from charging member handle 662 and extending perpendicularly from the inside of each charging member rail 664 towards each other, a stop interface 669 on an end of one of the charging member arms 666, and a pair of charging member return springs 670. Each of the charging member return springs 670 connects on one end to a charging member arm spring receiver 665 located on the outside of the charging member arm 666 and on the other end to the inside of meter housing 520. Charging member rails 664 slidingly support the guide hooks 639 of test strip platform 630. Each charging member arm 666 has a test strip platform rail 667 that slidingly engages with one of the platform side edges 635. Charging member arm 666 also includes a piston stop surface 668 that is used to arm drive piston 642 by engaging against charging contact stop surface 650. Piston stop surface 668 also stops the sliding movement of drive piston 642 when the armed lancet driver 640 is discharged. Fig. 12D is a perspective view of optional lancing depth control 620. Lancing depth control 620 is more fully illustrated in Figures 13A, B and C.

Fig. 13A illustrates a perspective view of lancing depth control 620. Lancing depth control 620 is considered optional since the lancet driver could be made for a single lancing depth. Lancing depth control 620 includes a detent side 622 and a depth gauge side 628. The preferred shape of lancing depth control 620 is a wheel having an outer peripheral surface 626. Outer peripheral surface 626 may include indicia 627 to indicate relative depth of penetration of the lance. Detent side 622 includes a plurality of extending tabs 624.

Fig. 13B shows depth gauge side 628 with a charging member interface surface 629. Charging member interface surface 629 is a gradually, recessing, radial surface that has a variable distance between the interface surface 629 and the back surface 628a of lancing depth control 620. Charging member interface surface 629 cooperatively engages with stop interface 669 of driver charging member 660 to set the lancing depth for lance 50.

Fig. 13C is a front view of lancing depth control 620. Extending tabs 624 are space along the detent side 622 to coincide with a pre-determined lancing depth for lance 50. The tab spaces 625 are important for engaging a detent 621 to temporarily lock the position of lancing depth control 620. Detent 621 may be a separate structure or may be integrally formed on the inside of meter housing 520. Extending tabs 624 are relatively rigid but have sufficient flexibility to allow the tabs to ride over detent 621 when lancing depth control 620 is re-positioned for a different lancing depth. Extending tabs 624 return to their original position when detent 621 enters a tab space 625 to temporarily set and temporarily lock the lancing depth control 620 to the desired lancing depth.

To determine the blood glucose level of a user, blood glucose test system 500 requires a lancet sensor strip assembly 510 and the handheld meter 520. To make the measurement, lancing depth control 620 is set to the preferred lancing depth. The charging handle 662 is pulled away from meter housing 520. This action causes the piston stop surface 668 of the charging member arms 666 to engage charging contact stop surface 650 of driver piston 642 pulling driver piston 642 toward an "armed" position and causing the piston drive spring 647 to compress against cross support 638a of test receiver platform 630. As driver piston 642 is pulled toward cross support 638a, driver piston holding surface 652 engages drive piston release 612 of lancet trigger 610 to hold driver piston 642 in the "armed" position. When the charging handle 662 is released after arming the driver piston 642, the charging member return springs 670 returns the charging handle 662 to its original position with the stop interface 669 resting against the charging member interface surface 629 of lancing depth control 620.

A lancet sensor test strip 510 is inserted into test strip port 524 of meter 520 and the protective lancet cover 1 is pulled away from the test strip. It should be noted that "arming" the driver piston 642 may be performed after the lancet sensor test strip 510 is inserted into test strip port 524. The end of lancet sensor test strip 510 is placed against the user's skin where the lancing is to be performed. Once in position, the user simply pushes the lancet trigger 610 releasing the driver piston 642. As the driver piston 642 moves, lancet driver surface 645 engages drive wings 145, 146 causing the lance 50 to extend out of lancet carrier 20 piercing the user's skin. Driver piston 642 stops when charging contact stop surface 650 hits piston stop surface 668 of charging member 660. Piston return springs 649 cause piston driver 642 to return to its discharged/resting position. Once drive wings 145, 146 are released, the spring action of sinuous portion 55 causes lance 50 to retract back into lancet carrier 20.

The user then removes the meter and strip from the wound site and massages or "milks" the wound site to generate sufficient sample for testing. The penetration depths of lance 50 are purposely selected for pain-free lancing. Because the penetration depth of lance 50 is so shallow, massaging or "milking" the wound site is necessary to obtain sufficient sample for testing. Once a sufficiently sized blood droplet appears, the meter and strip are returned to the wound site to allow the sample to enter the sample chamber of the sensor test strip. The meter then electrochemically determines the concentration of glucose in the blood from the blood sample. Once the measurement is complete, the disposable lancet sensor strip 510 is removed from meter 520 and discarded.

It should be understood that the blood glucose test system 500 may be configured to use anyone of the electrochemical methods used for determining the concentration of glucose in a sample. For example, these methods include amperometric, coulometric, potentiometric, voltammetric, and other electrochemical techniques.

## Claims

1. A lancet sensor assembly (10, 100) comprising:
an elongated carrier (20, 120, 120', 220) having a lancet member recess (21), a closed end (25, 225), an open end (27, 227), a first elongated side opening (23a, 123a, 223a), a peripheral support edge (23b, 223b) adjacent the top of said lancet member recess (21), and an assembly retaining mechanism (28, 228); and
a lancet member (40) having a lancet body (42), a lance (50) extending from a first lancet body end (43), a sinuous portion (55) extending from a second lancet body end (44) to a sinuous distal end (56), and a first drive wing (45, 145) extending outwardly from a first side (42a) of said lancet body (42), said lancet member (40) being disposed within said lancet member recess (21) wherein said first drive wing (45) is disposed through said first elongated side opening (23a, 123a), said lancet body (42) and said first drive wing (45) being slidingly engagable between a retracted position and an extended position wherein said distal end (56) of said sinuous portion (55) is anchored adjacent said closed end (25) of said elongated carrier (20, 120, 120', 220).

2. The lancet sensor assembly (10, 100) of Claim 1 wherein said elongated carrier (120', 220) has a first wing guard (129, 229) adjacent said first elongated side opening (23a, 123a, 223a).

3. The lancet sensor assembly (10, 100) of Claim 2 wherein said first wing guard (129, 229) includes a first wing guard slot to accommodate a lancet driver to effect translational movement of said drive wing (45, 145).

4. The lancet sensor assembly (10, 100) of Claims 1, 2, or 3 wherein said elongated carrier (20, 120, 120', 220) further includes a second elongated side opening (24a, 124a, 224a) on a second side (24, 224).

5. The lancet sensor assembly (10, 100) of Claim 4 wherein said lancet body (42) further includes a second drive wing (46, 146) extending outwardly from said second elongated side opening (24a, 124a, 224a) of said lancet body (42) and being disposed through said second elongated side opening (24a, 124a, 224a).

6. The lancet sensor assembly (10, 100) of Claim 5 wherein said elongated carrier (20, 120, 120', 220) further includes a second wing guard (130, 230) adjacent said second elongated side opening (24a, 124a, 224a).

7. The lancet sensor assembly (10, 100) of Claim 6 wherein said second wing guard (130, 230) includes a second wing guard slot to accommodate a lancet driver to effect translational movement of said second drive wing (46, 146).

8. The lancet sensor assembly (10, 100) of any one of Claims 1 to 7 further comprising a sensor strip (60) disposed on said peripheral support edge (23b, 223b) of said elongated carrier (20, 120, 120', 220) and retained in said elongated carrier (20, 120, 120', 220) by said assembly retaining mechanism (28, 228).

9. The lancet sensor assembly (10, 100) of Claim 8 further comprising a portable meter (520) having a lancet sensor assembly port (524) for receiving said lancet sensor assembly (10, 100), a lancet driver (640), and a lancet trigger (610), said lancet sensor assembly port (524) configured to electrically connect said portable meter (520) to said sensor strip (60) and to position said drive wing (45, 46, 145, 146) of said lancet member (40) for cooperation with said lancet driver (640).

10. The lancet sensor assembly (10, 100) of Claim 9 wherein said portable meter (520) further includes a detent surface (622) with a plurality of detent tabs (624) that extend axially outwardly from said detent surface forming a plurality of detent spaces (625) that are engagable with a detent (621) wherein each of said plurality of detent spaces (625) corresponds to a pre-determined lancing depth and a lancing depth control (620) having a charging member surface that cooperates with said lancet driver (640) to selectively limit the extended distance of said lancet driver (640) for controlling the lancing depth of said lance (50).

11. The lancet sensor assembly of Claim 9 wherein said lancet trigger (610) has a trigger body (611), a driver piston release (612) positioned near the base of said trigger body (611), a trigger arm (616) extending transversely from the top of said trigger body (611), and a user interface (614) located on said trigger arm (616) and spaced a pre-determined distance from the top of said trigger body (611) at a location that is along the central axis of said lancet sensor assembly port (524).

12. A method of using a lancet sensor assembly comprising:
engaging a lancet driver against a drive wing of a lancet assembly, said lancet assembly having a major portion of a lancet member slidably engaged within a lancet carrier having a lance connected to a first end of a lancet body of said lancet member wherein said lance is movable from a retracted position within said lancet carrier to an extended position outside said lancet carrier, said drive wing extending outwardly and perpendicularly from said lancet body, said major portion of said lancet member having a sinuous portion with a distal end restricted from movement by said lancet carrier;
moving said drive wing with said lancet driver a predetermined distance causing said lancet tip to move from said retracted position to said extended position; and
disengaging said lancet driver from said drive wing allowing said sinuous portion to move said lance from said extended position to said retracted position.

13. The method of Claim 12 further comprising arming said lancet driver before engaging said drive wing.

## Patentansprüche

1. Lanzettensensorbaugruppe (10, 100) umfassend:
einen länglichen Träger (20, 120, 120', 220) mit einer Lanzettenelementenausnehmung (21), einem geschlossenen Ende (25, 225), einem offenen Ende (27, 227), einer ersten länglichen Seitenöffnung (23a, 123a, 223a), einem peripheren Stützrand (23b, 223b) benachbart des Kopfs der Lanzettenelementenausnehmung (21) sowie einem Baugruppenhaltemechanismus (28, 228); und
ein Lanzettenelement (40) mit einem Lanzettenkörper (42), einer sich von einem ersten Lanzettenkörperende (43) erstreckenden Lanze (50), einem sich von einem zweiten Lanzettenkörperende (44) zu einem gewundenen distalen Ende (56) erstreckenden gewundenen Abschnitt (55) sowie einem ersten Antriebsflügel (45, 145), welcher sich von einer ersten Seite (42a) des Lanzettenkörpers (42) nach außen erstreckt, wobei das Lanzettenelement (40) in der Lanzettenelementenausnehmung (21) angeordnet ist, wobei der erste Antriebsflügel (45) durch die erste längliche Seitenöffnung (23a, 123a) hindurchragt, wobei der Lanzettenkörper (42) und der erste Antriebsflügel (45) zwischen einer zurückgezogenen Position und einer ausgefahrenen Position verschiebbar sind, wobei das distale Ende (56) des gewundenen Abschnitts (55) benachbart zu dem geschlossenen Ende (25) des länglichen Trägers (20, 120, 120', 220) verankert ist.

2. Lanzettensensorbaugruppe (10, 100) nach Anspruch 1, wobei der längliche Träger (120', 220) eine erste Flügelführung (129, 229) benachbart zu der ersten länglichen Seitenöffnung (23a, 123a, 223a) aufweist.

3. Lanzettensensorbaugruppe (10, 100) nach Anspruch 2, wobei die erste Flügelführung (129, 229) einen ersten Flügelführungsschlitz zur Aufnahme eines Lanzettentreibers aufweist, um eine translatorische Bewegung des Antriebsflügels (45, 145) zu bewirken.

4. Lanzettensensorbaugruppe (10, 100) nach Anspruch 1, 2 oder 3, wobei der längliche Träger (20, 120, 120', 220) ferner eine zweite längliche Seitenöffnung (24a, 124a, 224a) an einer zweiten Seite (24, 224) aufweist.

5. Lanzettensensorbaugruppe (10, 100) nach Anspruch 4, wobei der Lanzettenkörper (42) ferner einen zweiten Antriebsflügel (46, 146) aufweist, welcher sich von der zweiten länglichen Seitenöffnung (24a, 124a, 224a) des Lanzettenkörpers (42) nach außen erstreckt und durch die zweite längliche Seitenöffnung (24a, 124a, 224a) hindurchragt.

6. Lanzettensensorbaugruppe (10, 100) nach Anspruch 5, wobei der längliche Träger (20, 120, 120', 220) ferner eine zweite Flügelführung (130, 230) benachbart zu der zweiten länglichen Seitenöffnung (24a, 124a, 224a) aufweist.

7. Lanzettensensorbaugruppe (10, 100) nach Anspruch 6, wobei die zweite Flügelführung (130, 230) einen zweiten Flügelführungsschlitz zur Aufnahme eines Lanzettentreibers aufweist, um eine translatorische Bewegung des zweiten Antriebsflügels (46, 146) zu bewirken.

8. Lanzettensensorbaugruppe (10, 100) nach einem der Ansprüche 1 bis 7, ferner umfassend einen Sensorstreifen (60), welcher an dem peripheren Stützrand (23b, 223b) des länglichen Trägers (20, 120, 120', 220) angeordnet ist und in dem länglichen Träger (20, 120, 120', 220) durch den Baugruppenhaltemechanismus (28, 228) gehalten ist.

9. Lanzettensensorbaugruppe (10, 100) nach Anspruch 8, ferner umfassend ein tragbares Messgerät (520) mit einer Lanzettensensorbaugruppen-Öffnung (524) zur Aufnahme der Lanzettensensorbaugruppe (10, 100), einem Lanzettentreiber (460) und einem Lanzettenauslöser (610), wobei die Lanzettensensorbaugruppen-Öffnung (524) dazu eingerichtet ist, das tragbare Messgerät (520) mit dem Sensorstreifen (60) elektrisch zu verbinden und den Antriebsflügel (45, 46, 145, 146) des Lanzettenelements (40) zur Zusammenwirkung mit dem Lanzettentreiber (640) zu positionieren.

10. Lanzettensensorbaugruppe (10, 100) nach Anspruch 9, wobei das tragbare Messgerät (520) ferner eine Verrastungsfläche (622) mit einer Mehrzahl Rastzungen (624) aufweist, welche sich von der Verrastungsfläche axial nach außen erstrecken unter Bildung einer Mehrzahl Verrastungsräume (625), die mit einer Klinke (621) in Eingriff bringbar sind, wobei jeder der Mehrzahl Verrastungsräume (625) einer vorbestimmten Lanzierungstiefe entspricht, sowie eine Lanzierungstiefensteuerung (620) mit einer Ladelementenfläche aufweist, welche mit dem Lanzettentreiber (640) zusammenwirkt, um die ausgefahrene Strecke des Lanzettentreibers (640) zur Steuerung der Lanzierungstiefe der Lanze (50) selektiv zu begrenzen.

11. Lanzettensensorbaugruppe (10, 100) nach Anspruch 9, wobei der Lanzettenauslöser (610) einen Auslöserkörper (611), einen Antriebskolbenlöser (612), welcher nahe der Basis des Auslöserkörpers (611) angeordnet ist, einen Auslöserarm (616), welcher sich vom Kopf des Auslöserkörpers (611) quer erstreckt, sowie eine an dem Auslöserarm (616) gelegene Benutzerschnittstelle (614) aufweist, die einen vorbestimmten Abstand vom Kopf des Auslöserkörpers (611) an einer Stelle besitzt, welche längs der Mittelachse der Lanzettensensorbaugruppen-Öffnung (524) liegt.

12. Verfahren zur Verwendung einer Lanzettensensorbaugruppe, umfassend:
Ineingriffbringen eines Lanzettentreibers mit einem Antriebsflügel einer Lanzettenbaugruppe, wobei die Lanzettenbaugruppe einen Hauptteil eines Lanzettenelements in Schiebeeingriff in einem Lanzettenträger besitzt, der eine mit einem ersten Ende eines Lanzettenkörpers des Lanzettenelements verbundene Lanze aufweist, wobei die Lanze aus einer zurückgezogenen Position in dem Lanzettenträger in eine ausgefahrene Position außerhalb des Lanzettenträgers bewegbar ist, wobei der Antriebsflügel sich nach außen und senkrecht von dem Lanzettenkörper erstreckt, wobei der Hauptteil des Lanzettenelements einen gewundenen Abschnitt mit einem distalen Ende aufweist, das gegen Bewegung durch den Lanzettenträger gesichert ist,
Bewegen des Antriebsflügels mit dem Lanzettentreiber um eine vorbestimmte Strecke, wodurch eine Bewegung der Lanzettenspitze aus der zurückgezogenen Position in die ausgefahrene Position bewirkt wird, und
Außereingriffbringen des Lanzettentreibers mit dem Antriebsflügel, um dem gewundenen Abschnitt eine Bewegung der Lanze aus der ausgefahrenen Position in die zurückgezogene Position zu ermöglichen.

13. Verfahren nach Anspruch 12, ferner umfassend ein Armieren des Lanzettentreibers, bevor er in Eingriff mit dem Antriebsflügel gebracht wird.

## Revendications

1. Ensemble (10, 100) de capteur pour lancette comprenant :
un support allongé (20, 120, 120', 220) ayant un évidement (21) pour élément de lancette, une extrémité fermée (25, 225), une extrémité ouverte (27, 227), une première ouverture latérale allongée (23a, 123a, 223a), un bord support périphérique (23b, 223b) adjacent au sommet de l'évidement (21) pour élément de lancette, et un mécanisme (28, 228) de retenue d'ensemble ; et
un élément (40) de lancette ayant un corps (42) de lancette, une petite lance (50) s'étendant à partir d'une première extrémité (43) de corps de lancette, une partie sinueuse (55) s'étendant d'une deuxième extrémité (44) de corps de lancette jusqu'à une extrémité distale sinueuse (56), et une première ailette d'entraînement (45, 145) s'étendant vers l'extérieur à partir d'un premier côté (42a) dudit corps (42) de lancette, ledit élément (40) de lancette étant disposé à l'intérieur dudit évidement (21) pour élément de lancette dans lequel ladite première ailette d'entraînement (45) est disposée à travers ladite première ouverture latérale allongée (23a, 123a), ledit corps (42) de lancette et ladite première ailette d'entraînement (45) étant engageables de façon coulissante entre une position rétractée et une position étendue dans lequel ladite extrémité distale (56) de ladite partie sinueuse (55) est ancrée adjacente à ladite extrémité fermée (25) dudit support allongé (20, 120, 120', 220).

2. Ensemble (10, 100) de capteur pour lancette selon la revendication 1, dans lequel ledit support allongé (120', 220) a une protection (129, 229) de première ailette adjacente à ladite première ouverture latérale allongée (23a, 123a, 223a).

3. Ensemble (10, 100) de capteur pour lancette selon la revendication 2, dans lequel ladite protection (129, 229) de première ailette inclut une fente de protection de première ailette pour recevoir un entraîneur de lancette pour exécuter un mouvement de translation de ladite ailette d'entraînement (45, 145).

4. Ensemble (10, 100) de capteur pour lancette selon la revendication 1, 2 ou 3, dans lequel ledit support allongé (20, 120, 120', 220) inclut en outre une deuxième ouverture latérale allongée (24a, 124a, 224a) sur un deuxième côté (24, 224).

5. Ensemble (10, 100) de capteur pour lancette selon la revendication 4, dans lequel ledit corps (42) de lancette inclut en outre une deuxième ailette d'entraînement (46, 146) s'étendant vers l'extérieur à partir de ladite deuxième ouverture latérale allongée (24a, 124a, 224a) dudit corps (42) de lancette et étant disposée à travers ladite deuxième ouverture latérale allongée (24a, 124a, 224a).

6. Ensemble (10, 100) de capteur pour lancette selon la revendication 5, dans lequel ledit support allongé (20, 120, 120', 220) inclut en outre une protection (130, 230) de deuxième ailette adjacente à ladite deuxième ouverture latérale allongée (24a, 124a, 224a).

7. Ensemble (10, 100) de capteur pour lancette selon la revendication 6, dans lequel ladite protection (130, 230) de deuxième ailette inclut une fente de protection de deuxième ailette pour recevoir un entraîneur de lancette pour exécuter un mouvement de translation de ladite deuxième ailette d'entraînement (46, 146).

8. Ensemble (10, 100) de capteur pour lancette selon les revendications 1 à 7 comprenant en outre une bande (60) de capteur disposée sur ledit bord support périphérique (23b, 223b) dudit support allongé (20, 120, 120', 220) et retenue dans ledit support allongé (20, 120, 120', 220) par ledit mécanisme (28, 228) de retenue d'ensemble.

9. Ensemble (10, 100) de lancette selon les revendications 1 à 8 comprenant en outre un appareil de mesure portatif (520) ayant un orifice (524) pour ensemble de capteur pour lancette pour recevoir ledit ensemble (10, 100) de capteur pour lancette, un entraîneur (640) de lancette, et un déclencheur (610) de lancette, ledit orifice (524) pour ensemble de capteur pour lancette configuré pour connecter électriquement ledit appareil de mesure portatif (520) à ladite bande (60) de capteur et pour positionner ladite ailette d'entraînement (45, 46, 145, 146) dudit élément (40) de lancette pour une coopération avec ledit entraîneur (640) de lancette.

10. Ensemble (10, 100) de lancette selon la revendication 9, dans lequel ledit appareil de mesure portatif (520) inclut en outre une surface (622) de détente avec une pluralité d'onglets (624) de détente qui s'étendent axialement vers l'extérieur à partir de ladite surface de détente formant une pluralité d'espaces (625) de détente qui sont engageables avec une détente (621) dans lequel chacun de ladite pluralité d'espaces (625) de détente correspond à une profondeur de piquage prédéterminée et une commande (620) de profondeur de piquage ayant une surface d'élément de chargement qui coopère avec ledit entraîneur (640) de lancette pour limiter sélectivement la distance étendue dudit entraîneur (640) de lancette pour commander la profondeur de piquage de ladite petite lance (50).

11. Ensemble de lancette selon la revendication 9, dans lequel ledit déclencheur (610) de lancette a un corps (611) de déclencheur, un dispositif de libération (612) de piston d'entraîneur positionné à côté de la base dudit corps (611) de déclencheur, un bras (616) de déclencheur s'étendant transversalement à partir du sommet dudit corps (611) de déclencheur, et une interface utilisateur (614) située sur ledit bras (616) de déclencheur et espacée d'une distance prédéterminée du sommet dudit corps (611) de déclencheur en un emplacement qui est le long de l'axe central dudit orifice (524) pour ensemble de capteur pour lancette.

12. Procédé d'utilisation d'un ensemble de capteur pour lancette comprenant :
l'engagement d'un entraîneur de lancette contre une ailette d'entraînement d'un ensemble de lancette, ledit ensemble de lancette ayant une partie principale d'un élément de lancette engagé de façon coulissante à l'intérieur d'un support de lancette ayant une petite lance connectée à une première extrémité d'un corps de lancette dudit élément de lancette dans lequel ladite petite lance est mobile d'une position rétractée à l'intérieur dudit support de lancette jusqu'à une position étendue à l'extérieur dudit support de lancette, ladite ailette d'entraînement s'étendant vers l'extérieur et perpendiculairement à partir dudit corps de lancette, ladite partie principale dudit élément de lancette ayant une partie sinueuse avec une extrémité distale à mouvement restreint par ledit support de lancette ;
le déplacement de ladite ailette d'entraînement avec ledit entraîneur de lancette d'une distance prédéterminée, faisant que ladite pointe de lancette passe de ladite position rétractée à ladite position étendue ; et
le désengagement dudit entraîneur de lancette de ladite ailette d'entraînement, permettant que ladite partie sinueuse fasse passer ladite petite lance de ladite position étendue à ladite position rétractée.

13. Procédé selon la revendication 12, comprenant en outre l'armement dudit entraîneur de lancette avant l'engagement de ladite ailette d'entraînement.
